# EUROPÄISCHE PATENTANMELDUNG

(11) **EP 2 599 478 A1**
(43) Veröffentlichungstag der Anmeldung: **05.06.2013**
(21) Anmeldenummer: 11191254.9
(22) Anmeldetag: 30.11.2011
(51) Int. Cl.: A61K 9/70, A61K 31/00

(54) **Transdermales therapeutisches System zur Verabreichung von Fentanyl oder einem Analogstoff davon**

(71) Anmelder: Acino AG, 83714 Miesbach (DE)
(72) Erfinder: Schurad, Björn, 81667 München (DE); Schmitt, Sonja, 82008 Unterhaching (DE)
(74) Vertreter: Best, Michael

(57) **Zusammenfassung**

Erfindungsgemäß wird ein transdermales therapeutisches System zur Verabreichung von Fentanyl oder einem Analogstoff davon durch die Haut zur Verfügung gestellt, das eine haftklebende Matrixschicht aufweist, die ein Gemisch aus zwei Polyisobutylenen mit bestimmten Speichermodulen enthält.

## Beschreibung

Gegenstand der vorliegenden Anmeldung ist ein System zur transdermalen Verabreichung von Fentanyl oder einem Analogstoff davon für therapeutische Zwecke. Das transdermale therapeutische System (TDS, Wirkstoffpflaster) zeichnet sich durch eine hervorragende Kombination der für ein solches System relevanten Eigenschaften aus, insbesondere ausreichende Klebekraft und Hautverträglichkeit über einen langen Zeitraum, so dass eine Tragedauer des Systems von mindestens 3 Tagen gewährleistet ist, die Abwesenheit von kaltem Fluss, geringe Wirkstoffbeladung und ausgezeichnete Wirkstofffreisetzung.

Fentanyl und dessen Analogstoffe, insbesondere Alfentanil, Carfentanil, Lofentanil, Remifentanil, Sufentanil, Trefentanil und ähnliche Verbindungen sind wirksame synthetische Opiate. Fentanyl und dessen Analogstoffe sind hochwirksam und werden schnell metabolisiert. Problematisch bei diesen Verbindungen ist, dass sie einen relativ engen therapeutischen Index besitzen. Bei Überschreiten der Grenzwerte treten unerwünschte Nebeneffekte auf, insbesondere eine Beeinträchtigung der Atmung, die - wenn nicht geeignete Gegenmaßnahmen ergriffen werden - zum Tod führen kann. Die Wirkstoffe sind relativ teuer und es besteht ein sehr hohes Missbrauchsrisiko. Deshalb müssen Fentanylpflaster einerseits eine sehr präzise gesteuerte Freisetzung des Wirkstoffs gewährleisten und andererseits soll das Produkt so geschaffen sein, dass der Wirkstoff hieraus nicht leicht zu Missbrauchszwecken entfernt werden kann. Die Pflaster sind in der Regel für einen Einsatz von mindestens drei Tagen vorgesehen und müssen über diesen Zeitraum ausreichend auf der Haut kleben.

Ein Wirkstoffpflaster ist typischerweise eine kleine klebende Bandage, welche den abzugebenden Wirkstoff enthält. Diese Bandagen können verschiedene Formen und Größen haben. Der einfachste Typ ist ein Klebe-Monolith, welcher einen Wirkstoffvorrat (Reservoir) auf einem Träger umfasst. Das Reservoir wird typischerweise aus dem Wirkstoff in einem pharmazeutisch akzeptablen druckempfindlichen Klebstoff gebildet. Es kann aber auch aus einem nicht (oder schlecht) klebenden Material geformt sein, dessen Haut-Kontaktfläche mit einer dünnen Schicht eines geeigneten Klebstoffes versehen ist.

Komplexere Pflaster sind Mehrfachlaminate oder Pflaster mit Wirkstoffvorrat (der gegebenenfalls in einer Flüssigkeit gelöst vorliegen kann), in welchen zwischen dem Wirkstoffvorrat und dem die Haut kontaktierenden Klebstoff eine die Wirkstoff-Freisetzung steuernde Membran angeordnet sein kann. Diese Membran dient dazu, die Auswirkungen von Variationen der Hautdurchlässigkeit durch Herabsetzung der in vitro- und in vivo-Abgaberate des Wirkstoffs aus dem Pflaster zu kontrollieren und gegebenenfalls zu vermindern.

Ein Wirkstoffpflaster kann den Wirkstoff entweder vollständig gelöst im Vorrat enthalten oder der Vorrat kann einen Überschuss ungelösten Wirkstoffs enthalten (Depot-Pflaster). Die Anwesenheit von ungelöstem Wirkstoff oder anderen Bestandteilen in einem Pflaster kann bei der Lagerung sowie beim Gebrauch allerdings Stabilitäts- und andere Probleme aufwerfen. Eine Schwierigkeit besteht auch darin, dass sichergestellt werden muss, dass sich der Wirkstoff aus dem festen Depot ausreichend schnell nachlöst, um den abgegebenen Wirkstoff zu ersetzen. Wirkstoffpflaster, deren Reservoir feste Wirkstoffteilchen aufweist, werden im Stand der Technik daher häufig als nachteilig angesehen.

Aus dem Stand der Technik sind viele verschiedene transdermale Pflaster für die Verabreichung von Fentanyl bekannt. Die WO 02/074286 beschreibt ein transdermales Pflaster mit einem Reservoir enthaltend Fentanyl, wobei das Reservoir eine polymere Zusammensetzung, bevorzugt Polyacrylat, in einheitlichem Phasenzustand aufweist, die frei ist von nicht gelöstem Wirkstoff. Eine Übersättigung soll hier ausdrücklich vermieden werden.

Es gibt viele Versuche, Fentanylpflaster auch auf der Basis einer Matrixschicht aus Polyisobutylen herzustellen. Erste derartige Versuche werden bereits in dem Grundpatent zu Fentanylpflastern, dem US-A 4,588,580, beschrieben. Diese Druckschrift offenbart ein transdermales therapeutisches System mit einer Polyisobutylenmatrix und Mineralöl, die eine 2%ige Beladung von Fentanyl als ungelöstem Feststoff enthält. Das System weist in der Praxis jedoch Nachteile auf, und in der Folgezeit ging die Entwicklung daher von Polyisobutylenmatrices weg bzw. falls Polyisobutylenmatrices verwendet wurden, versuchte man, den Wirkstoff vollständig in der Polyisobutylenmatrix zu lösen.

Ein transdermales therapeutisches System mit einer Polyisobutylenmatrix ist in Roy et al., Journal of Pharmaceutical Sciences, Vol. 85, Nr. 5, Mai 1996, Seiten 491 bis 495 beschrieben. Es wird gezeigt, dass bei Konzentrationen von Fentanyl in der Polyisobutylenmatrix von mehr als 4% Wirkstoff ausfällt, und dies wurde offensichtlich von Roy et al. als negativ angesehen. Roy et al. schlägt für Pflaster mit geringer Wirkstoffbeladung an Fentanyl Polysilikonpflaster vor und keine Polyisobutylenpflaster.

In der US 2007/0009588 und der US 2006/0013865 werden dementsprechend Polyisobutylenmatrices vorgeschlagen, bei denen sorgfältig darauf zu achten ist, dass der Wirkstoff in der Polyisobutylenmatrix vollständig gelöst vorliegt. Das Auftreten von Kristallen in der Matrix wird als negativ angesehen. Die Polyisobutylenmatrices enthalten Polyisobutylene unterschiedlichen Molekulargewichts und ein Mineralöl, bei dem es sich bevorzugt um flüssiges Paraffin handelt oder ein Styrol-Isopren-Styrol Blockpolymer.

Die DE 198 37 902 offenbart transdermale therapeutische Systeme auf der Basis von Polyisobutylen, die insbesondere zur Verabreichung von Clonidin geeignet sind, unter den dort genannten Wirkstoffen findet sich aber auch Fentanyl. Beispiele für Fentanylpflaster finden sich in dieser Druckschrift nicht, ein Hinweis darauf, dass bei den dort offenbarten Pflastern der Wirkstoff als Feststoff vorhanden sein soll, findet sich in der Druckschrift ebenfalls nicht. Die Druckschrift enthält keine in vivo-Untersuchungen zur Freisetzung des Wirkstoffs aus den Pflastern. Die Polyisobutylenschicht dieser Pflaster enthält mindestens 5 Gew.-% eines Füllstoffs.

In der WO 2009/130039 werden transdermale therapeutische Systeme zur Verabreichung von Fentanyl oder einem Analogstoff davon beschrieben, bei denen auf einer Rückschicht eine Polyisobutylenschicht aufgebracht ist, die den Wirkstoff enthält und die einen Gehalt an Gelbildner von höchstens 4 Gew.-% aufweist. Mit diesen Pflastern soll eine längere und gleichmäßigere Freisetzung erzielt werden, als mit den bekannten Fentanylpflastern.

Die EP 0 272 987 offenbart ebenfalls ein Pflaster zur Verabreichung von Wirkstoffen, wobei eine besonders ausgestaltete Trägerschicht als relevant angesehen wird. In einem Beispiel der Druckschrift wird ein Fentanylpflaster offenbart, das zwei Wirkstoffschichten enthält, wobei die eine Wirkstoffschicht eine Polyisobutylenschicht, die andere eine Polydimethylsiloxanschicht darstellt. Die Druckschrift offenbart, dass der Wirkstofffluss aus der Polyisobutylenschicht wesentlich niedriger ist, als aus der Polydimethylsiloxanschicht.

Die WO 2011/029948 offenbart ein transdermales therapeutisches System zur Verabreichung von Fentanyl oder einem Analogstoff des Fentanyls durch die Haut bei der die Matrixschicht aus zwei Schichten besteht, die jeweils ein Polyisobutylen mit einer anderen Zusammensetzung enthalten. Der Aufbau der Pflaster ist durch die Mehrfachbeschichtung relativ komplex.

Die US 2011/0020426 offenbart ein Verfahren zur Herstellung einer Klebstoffzusammensetzung aus einem Polyisobutylen mit hohem Molekulargewicht und einem Polyisobutylen mit geringerem Molekulargewicht. Diese Klebstoffzusammensetzung ist für Pflaster zur Verabreichung von Wirkstoff geeignet. Die Wirkstoffe sind nicht besonders eingeschränkt und Fentanyl wird als ein Wirkstoff aus einer langen Liste von Wirkstoffen offenbart. Keines der Beispiele betrifft ein Fentanylpflaster.

Die EP 1 625 845 offenbart ein transdermales therapeutisches System für die Verabreichung von Fentanyl aus einer Klebstoffschicht aus zwei Arten von Polyisobutylen mit unterschiedlichem Molekulargewicht. Die Klebstoffschicht enthält weiterhin noch einen Klebrigmacher und eine organische Flüssigkeit, bei der es sich um Polybuten handeln kann. Wie in der US 2007/0009588 und der US 2006/0013865 sieht es die EP 1 625 845 als wesentlich an, dass das Fentanyl in der Klebstoffschicht vollständig gelöst ist. Dementsprechend werden der Klebrigmacher und die organische Flüssigkeit ausgewählt, um eine vollständige Lösung des Fentanyls in der Klebstoffschicht zu gewährleisten. Bei der organischen Flüssigkeit handelt es sich bevorzugt um ein Gemisch aus einem Fettsäurealkylester und einem verzweigten langkettigen Alkohol, da in diesem Gemisch die Löslichkeit des Fentanyls am Stärksten ist.

Es ist eine ausgesprochen anspruchsvolle Aufgabe, ein transdermales therapeutisches System für die Verabreichung von Fentanyl oder einem Analogstoff davon zur Verfügung zu stellen, das alle praxisrelevanten Eigenschaften vorteilhaft verbindet. Ein derartiges transdermales therapeutisches System muss nicht nur über die gesamte Anwendungsdauer eine ausreichende Wirkstofffreisetzung gewährleisten, sondern darüber hinaus auch eine ausreichende Klebekraft und Hautverträglichkeit damit die für Fentanyl und Analogstoffe davon übliche Anwendungsdauer von typischerweise drei Tagen gewährleistet ist. Ferner darf bei dem transdermalen therapeutischen System kein nennenswerter kalter Fluss auftreten. Bei einem solchen kalten Fluss wird das transdermale therapeutische System während der Lagerung praktisch unbrauchbar. Schließlich sollte das Pflaster auch noch einfach und kostengünstig herzustellen sein und mit möglichst wenig Wirkstoffbeladung auskommen.

Die im Stand der Technik vorgeschlagenen Pflaster auf Polyisobutylenbasis erfüllen Teilaspekte der vorstehenden erwünschten Eigenschaften. Beispielsweise zeigt das in der EP 1 625 845 beschriebene Pflaster mit einer Matrixschicht aus zwei Polyisobutylenen mit unterschiedlichem Molekulargewicht eine zufriedenstellende Freisetzung des Wirkstoffs, allerdings ist die Klebleistung der Pflaster nicht zufriedenstellend. Bei nicht hinreichender Klebeleistung des Pflasters ist selbstverständlich eine Anwendung über einen längeren Zeitraum, z.B. von 3 Tagen oder mehr, nicht zuverlässig möglich. Auch ist die Menge an Wirkstoff, die in das Pflaster eingebracht wird, für ein Mehrtagespflaster zur Behandlung von starken Schmerzen zu gering. In der WO 2011/029948 wurde ein Weg gefunden, die Klebeeigenschaften der Fentanylpflaster auf Polyisobutylenbasis zu verbessern und gleichzeitig eine ausgezeichnete Wirkstofffreisetzung zu erzielen. In die Pflaster kann eine Menge an Wirkstoff eingebracht werden, die auch zur Behandlung starker Schmerzen über mehrere Tage ausreichend ist. Allerdings neigen die dort offenbarten Pflaster dazu, kalten Fluss zu zeigen und der Aufbau der Pflaster mit zwei übereinander angeordneten haftklebenden Schichten aus verschiedenen Polyisobutylenen ist relativ komplex was die Herstellung des Pflasters aufwendig und teuer macht.

Es besteht daher weiterhin Bedarf nach einem Pflaster auf der Basis von Polyisobutylenen zur Verabreichung von Fentanyl oder einem Analogstoff davon, das die vorstehend genannte vorteilhafte Kombination an Eigenschaften aufweist, das also nicht nur eine hervorragende Freisetzung des Wirkstoffs sondern auch sehr gute Klebeeigenschaften und hervorragenden Tragekomfort (jeweils von drei Tagen oder mehr), zur Verfügung stellt, dabei bei der Lagerung keinen kalten Fluss zeigt und das leicht und kostengünstig mit geringem Wirkstoffeinsatz hergestellt werden kann.

Die Erfinder der vorliegenden Anmeldung haben nunmehr überraschend gefunden, dass die Temperaturabhängigkeit des viskoelastischen Verhaltens der Polyisobutylene der Matrixschicht eines transdermalen therapeutischen Systems von entscheidender Bedeutung für die relevanten Eigenschaften des transdermalen therapeutischen Systems ist, insbesondere für die Kombination der Eigenschaften a) gute Klebeeigenschaft, b) Minimierung von kaltem Fluss und c) hohe Wirkstofffreisetzung über mehrere Tage. Es wurde gefunden, dass transdermale therapeutische Systeme, bei denen die Matrixschicht aus Polyisobutylenen aufgebaut ist, deren viskoelastische Eigenschaften eine bestimmte Temperaturabhängigkeit zeigen, die Kombination der vorstehend genannten hervorragenden Eigenschaften aufweisen, insbesondere wenn die Matrixschicht noch einen Permeationsverstärker und einen Klebrigmacher enthält. Überraschenderweise wurde gefunden, dass insbesondere die Temperaturabhängigkeit des Speichermoduls der in der Matrixschicht vorhandenen Polyisobutylene eine entscheidende Rolle spielt, um kalten Fluss zu vermeiden, gleichzeitig eine hohe Klebkraft bei sehr guter Hautverträglichkeit über einen Zeitraum von mindestens drei Tagen zur Verfügung zu stellen und trotzdem eine ausreichende Freisetzung des Wirkstoffs zu gewährleisten. Ein komplexer mehrschichtiger Aufbau, wie er beispielsweise in der WO 2011/029948 verlangt wird, ist bei den erfindungsgemäßen transdermalen therapeutischen Systemen nicht erforderlich.

Die vorliegende Anmeldung betrifft damit ein transdermales therapeutisches System zur Verabreichung eines Wirkstoffs durch die Haut, umfassend
a) eine Rückschicht,
b) eine haftklebende Matrixschicht, die den Wirkstoff enthält, und
c) eine Abziehschicht (Release-Liner),
wobei der Wirkstoff Fentanyl oder ein Analogstoff des Fentanyls ausgewählt aus Alfentanil, Lorfentanil, Lofentanil, Remifentanil und Trefentanil oder ein Salz eines dieser Wirkstoffe ist und
wobei die Matrixschicht als haftklebendes Polymer ein Gemisch aus einem Polyisobutylen A und einem Polyisobutylen B enthält,
wobei das Polyisobutylen A ein Speichermodul G' aufweist, dessen Wert im Temperaturbereich von 10°C bis 40°C im Wesentlichen konstant ist und
wobei das Polyisobutylen B ein Speichermodul G' aufweist, dessen Wert im Temperaturbereich von 10°C bis 40°C mit steigender Temperatur kontinuierlich abnimmt,
wobei das Speichermodul G' im linearen viskoelastischen Bereich bei einer Frequenz von 10 rad/sec unter Verwendung eines Rheometers mit paralleler Plattengeometrie und parallelen Platten gemessen wird und
wobei die haftklebende Matrixschicht ungelösten Wirkstoff in Form von Wirkstoffpartikeln enthält.

Das erfindungsgemäße transdermale therapeutische System enthält als Wirkstoff Fentanyl oder einen Analogstoff des Fentanyls ausgewählt aus Alfentanil, Carfentanil, Lofentanil, Remifentanil und Trefentanil oder ein Salz einer dieser Wirkstoffe. Am meisten bevorzugt ist der Wirkstoff Fentanyl. Die Erfindung wird im Folgenden im Wesentlichen anhand des Wirkstoffs Fentanyl erläutert. Die Ausführungen gelten aber entsprechend auch für die angegebenen Analogstoffe des Fentanyls.

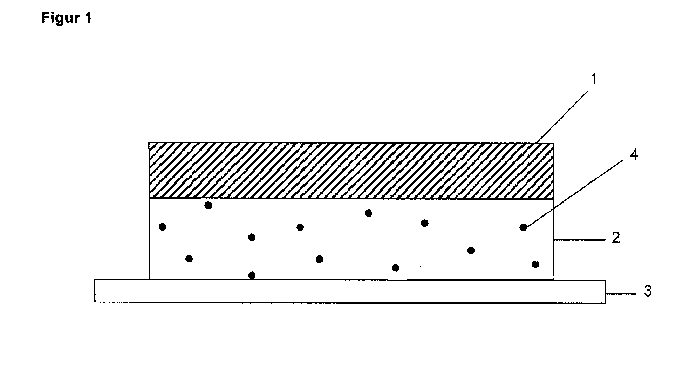

Der bevorzugte Aufbau des erfindungsgemäßen transdermalen therapeutischen Systems in seiner einfachsten Ausgestaltung ist in Figur 1 gezeigt. Auf dem bei der Anwendung der Haut gegenüber befindlichen Ende des transdermalen therapeutischen Systems befindet sich die Rückschicht (1). An der der Haut beim Gebrauch zugewandten Seite der Rückschicht (1) befindet sich die haftklebende Matrixschicht (2), die auch als Reservoir bezeichnet wird. Bei dem bevorzugten erfindungsgemäßen transdermalen therapeutischen System handelt es sich um ein Suspensionspflaster, d.h. der Wirkstoff ist teilweise ungelöst in der haftklebenden Matrixschicht suspendiert. In Figur 1 sind die Wirkstoffteilchen (4) gezeigt.

Wesentlich für die bevorzugten erfindungsgemäßen transdermalen therapeutischen Systeme ist, dass die haftklebende Matrixschicht so viel Wirkstoff enthält, dass ein Teil des Wirkstoffs in ungelöster Form, also als Wirkstoffpartikel, vorliegt.

Bei der Herstellung des erfindungsgemäßen transdermalen therapeutischen Systems wird der Wirkstoff bevorzugt in mikronisierter Form, bei der mehr als 90% der Partikel kleiner als 50 µm, bevorzugt kleiner 25 µm, eingesetzt. Auch in der Matrixschicht des transdermalen therapeutischen Systems liegt der Wirkstoff in mikronisierter Form vor, allerdings können sich durch Umlagerungsreaktionen bei der Herstellung und Lagerung des transdermalen therapeutischen Systems Abweichungen der Teilchengröße ergeben. Auch innerhalb des transdermalen therapeutischen Systems sind vorzugsweise mehr als 90% der Wirkstoffpartikel kleiner als 100 µm, stärker bevorzugt kleiner als 50 µm und insbesondere kleiner als 25 µm. Zur Herstellung des erfindungsgemäßen transdermalen therapeutischen Systems wird bevorzugt mikronisiertes Fentanyl mit einer mittleren Teilchengröße von 1 µm oder mehr, stärker bevorzugt 2 µm oder mehr, eingesetzt. Bei dem eingesetzten Fentanyl sind bevorzugt mehr als 90% der Partikel kleiner 25 µm. Diese Teilchengrößen finden sich bevorzugt ebenfalls in dem fertig hergestellten transdermalen therapeutischen System. In der Matrixschicht des transdermalen therapeutischen Systems kann die Teilchengröße und die Teilchengrößenverteilung der Wirkstoffteilchen am besten durch übliche Lichtmikroskopie bestimmt werden. Die Auswertung erfolgt über übliche Computerprogramme (Bildverarbeitungssysteme), die in der Regel auf die verwendeten Mikroskope abgestimmt sind. Die Teilchengröße bezieht sich auf den Teilchendurchmesser, sofern nichts anderes angegeben oder offensichtlich ist.

Als Ausgangsmaterial für das mikronisierte Fentanyl wird handelsübliches Fentanyl eingesetzt, das an sich für die klinische Anwendung geeignet ist. Derartiges Fentanyl hat üblicherweise eine Verteilung der Partikelgröße dergestalt, dass 90% der Partikel kleiner sind als 2500 µm. Vorzugsweise sind etwa 90% der Partikel kleiner als etwa 1000 µm und/oder mehr bevorzugt 50% der Partikel kleiner als etwa 100 µm.

Erfindungsgemäß kann jedes bekannte Mikronisierungsverfahren verwendet werden, das die gewünschte Teilchengröße zur Verfügung stellt. Bevorzugt wird Fentanyl eingesetzt, das mittels einer üblichen "jet mill" mikronisiert wurde, z.B. einer jet mill vom Typ AS der Hosokawa Alpine AG.

Durch das erfindungsgemäß eingesetzte Mikronisierungsverfahren wird die Größe der Fentanylpartikel bevorzugt so eingestellt, dass die mittlere Teilchengröße in den vorstehend angegebenen Bereichen liegt. Bevorzugt ist ebenfalls, dass mehr als 90% der Partikel kleiner sind als 50 µm, mehr bevorzugt kleiner als 25 µm.

Für die Bestimmung der Teilchengröße bzw. der Teilchengrößenverteilung des Wirkstoffs gibt es verschiedene Verfahren, beispielsweise Lichtbeugungsverfahren (Laserdiffraktometrie), wie sie in den Geräten der Firma Malvern Instruments, z.B. dem "Malvern MasterSizer X", verwendet werden, mechanische Siebrüttelverfahren, wie sie die Firma FMC zur Bestimmung der Korngrößenverteilung ihrer AVICEL PH^{®}-Produkte verwendet, oder auch "air jet"-Siebanalysen, die beispielsweise mit einem ALPINA^{®}-"air jet" Modell 200 ausgeführt werden können.

Sofern nicht anders angegeben, werden die (mittleren) Teilchengrößen bzw. Teilchengrößenverteilungen mit dem Verfahren Laserdiffraktometrie bestimmt, beispielweise mit dem Gerät Mastersizer 2000 von Malvern.

Definiert man den Wirkstoff über die Angabe der mittleren Teilchengröße und die Teilchengrößenverteilung, weist der erfindungsgemäß eingesetzte mikronisierte Wirkstoff bevorzugt eine mittlere Teilchengröße von 20 µm oder weniger auf, und es ist bevorzugt, dass der Wirkstoff eine Korngrößenverteilung (Teilchengrößenverteilung) hat, bei der weniger als 10% der Teilchen eine Größe von 30 µm oder darüber haben und weniger als 10% der Teilchen eine Größe von 1 µm oder darunter haben.

Das erfindungsgemäße transdermale therapeutische System weist ebenfalls in der Regel eine Abdeckschicht (3) auf, die die haftklebende Matrixschicht bedeckt und die vor Gebrauch und Anwendung des Pflasters zu entfernen ist.

In der am stärksten bevorzugten Ausführungsform besteht das erfindungsgemäße transdermale therapeutische System ausschließlich aus der Rückschicht (1) und der haftklebenden Matrixschicht (2), die den Wirkstoff enthält und der vor Gebrauch zu entfernenden Abdeckschicht (3).

Es ist auch möglich, dass das transdermale therapeutische System auf der haftklebenden Matrixschicht (2) noch eine zusätzliche Klebstoffschicht oder ein Overtape aufweist die die Haftung des erfindungsgemäßen transdermalen therapeutischen Systems auf der Haut eines Patienten verbessern soll. Bei einem Overtape handelt es sich um eine zusätzliche Klebstoffschicht, die größter ist als die wirkstoffhaltige Schicht bzw. das eigentliche TDS und somit die Haftung verbessert. Erfindungsgemäß wurde aber gefunden, dass eine solche zusätzliche Klebstoffschicht bzw. ein Overtape bei dem erfindungsgemäßen transdermalen therapeutischen System nicht erforderlich ist und daher ist erfindungsgemäß ein transdermales therapeutisches System bevorzugt, das eine solche zusätzliche Klebstoffschicht bzw. ein Overtape nicht enthält. Eine derartige Klebstoffschicht kann aus verschiedenen an sich bekannten Haftklebstoffen bestehen, wie z.B. ebenfalls Polyisobutylen, Polysilikon oder Polyacrylat.

Ebenfalls ist es möglich, dass auf der haftklebenden Matrixschicht (2) noch eine Membran aufgebracht ist, die die Freisetzung des Wirkstoffs kontrolliert. Erfindungsgemäß wurde wiederum gefunden, dass bei dem erfindungsgemäßen Aufbau des transdermalen therapeutischen Systems eine solche Membran nicht erforderlich ist, sie kann aber selbstverständlich (beispielsweise zur Erhöhung der Sicherheit) aufgebracht werden. Geeignete Membranen sind im Stand der Technik bekannt und können beispielsweise auf der Basis von Polypropylen oder Polyethylenvinylacetat aufgebaut sein, beispielsweise kann es sich bei der Membran und eine mikroporöse Polypropylenfolie handeln. Geeignete Membranen sind in der WO 2009/130039 auf Seite 5 offenbart, deren Offenbarung insoweit hier durch Bezugnahme aufgenommen wird.

Da bei den erfindungsgemäßen transdermalen therapeutischen Systemen eine derartige Membran aber nicht zwingend erforderlich ist, weisen die erfindungsgemäßen transdermalen therapeutischen Systeme bevorzugt keine solche Membran auf. Falls eine derartige Membran dennoch vorhanden ist, befindet sich auf ihr noch eine Klebstoffschicht damit das transdermale therapeutische System auf der Haut haftet.

In der besonders bevorzugten Ausführungsform der erfindungsgemäßen transdermalen therapeutischen Systeme bestehen daher die transdermalen therapeutischen Systeme ausschließlich aus der Rückschicht (1), der haftklebenden Matrixschicht (2), die den Wirkstoff enthält und der Abziehschicht (3), die vor Gebrauch des transdermalen therapeutischen Systems zu entfernen ist und zusätzliche Klebstoffschichten oder eine die Abgabe des Wirkstoffs steuernde Membran sind nicht vorhanden.

Die Rückschicht (1) des transdermalen therapeutischen Systems ist in einer bevorzugten Ausführungsform okklusiv (also abschließend). Derartige Rückschichten sind im Stand der Technik bekannt und können beispielsweise aus Polyolefinen, insbesondere Polyethylen, aus Polyestern oder Polyurethanen bestehen. Auch Schichten, die mehrere verschiedene Polymere übereinander angeordnet beinhalten, sind vorteilhaft als Rückschichten in den erfindungsgemäßen transdermalen therapeutischen Systemen einsetzbar. Ein geeignetes Material für die Rückschicht ist beispielsweise ein Polyolefin, das von der Firma Mylan Technologies Inc. unter der Bezeichnung Mediflex^{®} 1000 vertrieben wird. Andere geeignete Materialien umfassen Cellophan, Celluloseacetat, Ethylcellulose, mit Weichmachern versehene Vinylacetatvinylchloridcopolymere, Ethylenvinylacetatcopolymere, Polyethylenterephthalat, Nylon, Polyethylen, Polypropylen, Polyvinylidenchlorid, Ethylenmethacrylatcopolymere, Papier, das gegebenenfalls beschichtet sein kann, textile Gewebe und Polyesterfolien wie Polyethylenterephthalatfolien. Besonders bevorzugt sind auch Aluminiumfolien und Polymer-Metall-Kompositmaterialien. Die Dicke der Rückschicht ist wie im Stand der Technik üblich z.B. im Bereich von 10 µm bis 80 µm, und in den Beispielen wurde eine Rückschicht mit einer nominellen Dicke von etwa 10-55 µm eingesetzt.

Auf der Rückschicht des Pflasters kann sich noch eine Abdeckschicht befinden, wie es im Stand der Technik bekannt ist. Die Abdeckschicht liegt bevorzugt lose auf der Rückschicht auf und wird durch elektrostatische Kräfte gehalten. Derartige Abdeckschichten sind beispielsweise in der EP 1 097 090 beschrieben, auf die insoweit vollinhaltlich Bezug genommen wird. Die Abdeckschicht ist zumindest auf der auf der Rückschicht aufliegenden Seite antihaftbeschichtet, z.B. silikonisiert oder fluoriert.

Die auf der haftklebenden Matrixschicht aufliegende Abziehschicht (3) wird üblicherweise auch als Release-Liner bezeichnet. Sie wird üblicherweise auf der haftklebenden Matrixschicht aufgebracht, um ein Verkleben der haftklebenden Matrixschicht beispielsweise mit der Verpackung zu verhindern und vor Gebrauch des transdermalen therapeutischen Systems entfernt. Die Abziehschicht wird bevorzugt aus polymerem Material hergestellt, das gegebenenfalls auch metallisiert sein kann. Beispiele für bevorzugt eingesetzte polymere Materialien sind Polyurethane, Polyvinylacetat, Polyvinylidenchlorid, Polypropylen, Polycarbonat, Polystyrol, Polyethylen, Polyethylenterephthalat, Polybutylenterephthalat sowie gegebenenfalls mit entsprechenden Polymeren oberflächenbeschichtete Papiere. Bevorzugter ist die Abziehschicht einseitig oder beidseitig fluorpolymerbeschichtet oder silikonisiert. Bevorzugt sind handelsübliche fluorpolymerbeschichtete oder silikonisierte Polyesterfolien wie die einseitig silikonisierten Handelsprodukte Primeliner 75 µm oder 100 µm (Firma Loparex, NL) sowie die einseitig fluorpolymerbeschichteten Handelsprodukte Scotchpack 1022 oder Scotchpack 9742 der Firma 3M.

Erfindungsgemäß wesentlich ist die Zusammensetzung der haftklebenden Matrixschicht, die den Wirkstoff enthält. Die für die Matrixschicht verwendeten Polyisobutylene müssen sehr sorgfältig ausgewählt werden, um eine ausreichende Wirkstofffreisetzung zu gewährleisten, gleichzeitig aber auch eine ausreichende Haftung ohne nennenswerte Hautreizungen auf der Haut, über einen Zeitraum von mindestens drei Tagen, und Minimierung von kaltem Fluss auch während der Lagerung sicherzustellen. Die Auswahl der entsprechenden Polymere wird dadurch erschwert, dass die transdermalen therapeutischen Systeme bei einer anderen Temperatur gelagert werden als sie angewendet werden. Die transdermalen therapeutischen Systeme werden bei Raumtemperatur gelagert und dann mit der haftklebenden Schicht auf die menschliche Haut aufgebracht, die eine Temperatur von etwa 32°C aufweist. Bei dieser Temperatur müssen die transdermalen therapeutischen Systeme dann ausreichend haften und eine ausreichende Wirkstofffreisetzung zeigen.

Überraschenderweise wurden erfindungsgemäß transdermale therapeutische Systeme gefunden, bei denen der kalte Fluss insbesondere auch bei der Anwendungstemperatur (Hauttemperatur = 32°C) ausgesprochen niedrig ist, und mit steigender Temperatur fällt. Die erfindungsgemäßen transdermalen therapeutischen Systeme weisen aber auch bei den Temperaturen, bei denen häufig eine Lagerung der transdermalen therapeutischen Systeme stattfindet, einen besonders niedrigen kalten Fluss auf.

Erfindungsgemäß wurde nunmehr überraschend gefunden, dass die Temperaturabhängigkeit des Speichermoduls eines Polyisobutylens einen entscheidenden Einfluss auf die Eigenschaften eines transdermalen therapeutischen Systems hat. Insbesondere wurde gefunden, dass eine hervorragende Wirkstofffreisetzung bei gleichzeitig exzellenter und langanhaltender Haftung der haftklebenden Matrixschicht auf der Haut und weitgehender Abwesenheit von kaltem Fluss bei der Lagerung des transdermalen therapeutischen Systems erzielt werden kann, wenn als haftklebendes Matrixmaterial ein Gemisch aus zwei Polyisobutylenen verwendet wird, von denen eines, das hier als Polyisobutylen (A) bezeichnet wird im Temperaturbereich von 10 bis 40°C, vorzugsweise von 10 bis 60°C und insbesondere von 0 bis 80°C praktisch keine Temperaturabhängigkeit des Speichermoduls G' zeigt, während das zweite Polyisobutylen, das hier als Polyisobutylen (B) bezeichnet wird in diesem Temperaturbereich ein mit steigender Temperatur kontinuierlich fallendes Speichermodul G' aufweist. Aufgrund dieser überraschenden Erkenntnis wurde dann das erfindungsgemäße transdermale therapeutische System entwickelt.

Das Speichermodul G' sowie das Verlustmodul G" sind dem Fachmann bekannte Parameter, die einen viskoelastischen Stoff kennzeichnen. Man geht davon aus, dass die Stärke des elastischen Anteils eines viskoelastischen Polymers durch das Speichermodul G' und die Stärke des viskosen Anteils eines viskoelastischen Polymers durch das Verlustmodul G" beschrieben wird. Der Quotient aus Verlustmodul G" und Speichermodul G' G"/G' wird als Verlusttangens tan δ bezeichnet. Die Frequenz bei der Messung von G' und G" wird in der vorliegenden Erfindung bei 10 rad/Sekunde konstant gehalten. Im Rahmen der vorliegenden Anmeldung wurde das Speichermodul G' und das Verlustmodul G" mit einer Vorrichtung Rheometrics RDA-III der Firma TA-Instruments, Newcastle, Delaware, USA bestimmt (parallele Platten, 8 mm Durchmesser, Abstand 2-2,5 mm). Die Bestimmung von G' und G" erfolgt bei niedrigen Auslenkspannungen bei der Messung im linearen viskoelastischen Bereich, und ist daher von der Spannung unabhängig. Im Übrigen werden G' und G" nach den Vorschriften der DIN EN ISO 6721-1:2011 bestimmt.

Polyisobutylene sind im Stand der Technik bekannt und werden von einer ganzen Reihe von Firmen angeboten, unter anderem auch in Form von Lösungen. Die Polyisobutylene können übliche Stabilisatoren oder Konservierungsmittel enthalten. Der Ausdruck "Polyisobutylene" wie er im Rahmen dieser Anmeldung verwendet wird, umfasst Polyisobutylene, die derartige übliche Konservierungsmittel und/oder Stabilisatoren enthalten oder nicht enthalten.

Das Speichermodul G' ist neben der Molekulargewichtsverteilung ein typischer Parameter zur Charakterisierung der Polyisobutylene. Polyisobutylene, die durch bestimmte Speichermodule G' gekennzeichnet sind, sind kommerziell erhältlich. Die in den Beispielen der vorliegenden Anmeldung verwendeten Polyisobutylenlösungen wurden von der Firma Henkel Corporation, Bridgewater, USA erhalten, es können aber selbstverständlich auch Konkurrenzprodukte verwendet werden. Die Angabe des gewünschten Verlaufs des Speichermoduls ist für den Hersteller der Polyisobutylene ausreichend, um ein entsprechendes Polyisobutylen zur Verfügung zu stellen. Selbstverständlich wird das Speichermodul an dem Polyisobutylen nach Entfernen der Lösemittel bestimmt.

Das erfindungsgemäße transdermale therapeutische System weist eine haftklebende Matrixschicht auf, die ein Gemisch aus einem Polyisobutylen A und einem Polyisobutylen B enthält.

Das Polyisobutylen A ist durch sein Speichermodul G' gekennzeichnet, das im Temperaturbereich von 10 bis 40°C, vorzugsweise von 10 bis 60°C und insbesondere von 0 bis 80°C im Wesentlichen konstant ist, das heißt das Speichermodul G' weist in diesem Temperaturbereich ein Plateau auf. Unter "im Wesentlichen konstant" wird erfindungsgemäß verstanden, dass sich bei der Auftragung der Werte des Speichermoduls G' (als Logarithmus) gegen die Temperatur im Temperaturbereich von zumindest 10 bis 40°C, vorzugsweise von 10 bis 60°C und insbesondere von 0 bis 80°C ein flaches Plateau zeigt. In einer bevorzugten Ausführungsform des erfindungsgemäßen transdermalen therapeutischen Systems bedeutet "im Wesentlichen konstant", dass in dem angegebenen Temperaturbereich keiner der Werte für das Speichermodul G' mehr als 50% (absolut, d.h. nicht logarithmisch), stärker bevorzugt mehr als 25% (absolut) und insbesondere mehr als 15% (absolut) von dem Wert des Speichermoduls bei 40°C, nach unten abweicht und dass keiner der Werte für das Speichermodul G' mehr als 100% (absolut), bevorzugt mehr als 50% (absolut), stärker bevorzugt mehr als 25% (absolut) nach oben abweicht.

Der Absolutwert des Plateaus im Bereich von 10 bis 40°C, vorzugsweise von 10 bis 60°C und insbesondere von 0 bis 80°C liegt für das Polyisobutylen A vorzugsweise in dem Bereich von 5 × 10⁴ Pascal bis 5 × 10⁶ Pascal, insbesondere im Bereich von 10⁵ bis 10⁶ Pascal und am stärksten bevorzugt im Bereich von 10⁵ Pascal bis 5 × 10⁵ Pascal. Die Molekulargewichtsverteilung des Polyisobutylens A ist für die vorliegende Erfindung nicht relevant, so lange die Werte für das Speichermodul G' eingehalten werden.

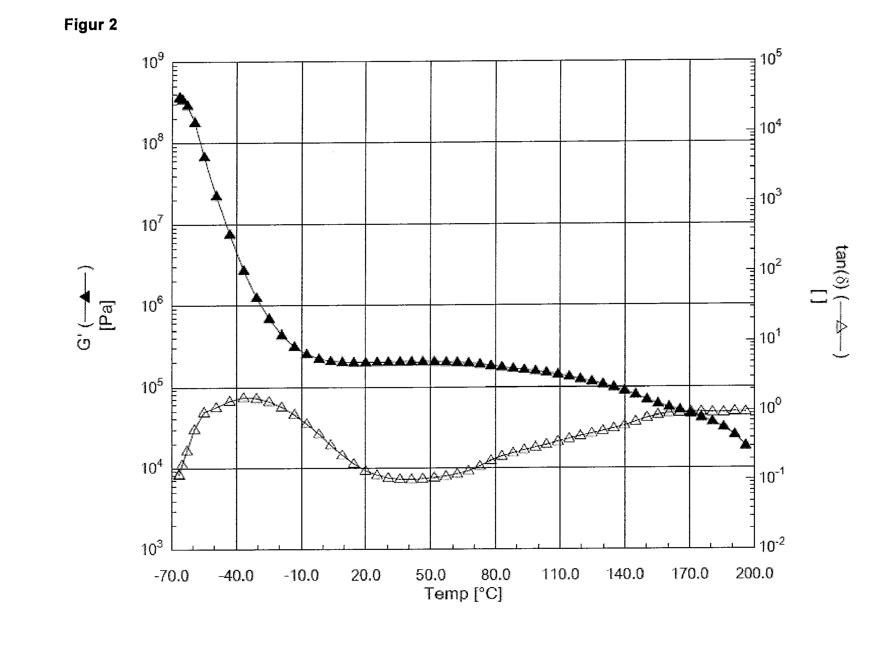

Das Polyisobutylen A kann beispielsweise durch Mischen von zwei oder mehr Polyisobutylenen hergestellt werden, so dass sich ein Polyisobutylen mit dem gewünschten Verlauf für das Speichermodul ergibt. Vorzugsweise handelt es sich bei dem Polyisobutylen A jedoch um ein einzelnes Polyisobutylen. Ein Beispiel für ein geeignetes Polyisobutylen A ist das Produkt Durotak 87-625A der Firma Henkel Corporation, Brigewater, USA. Der Verlauf des Speichermoduls G' (zusammen mit dem Verlauf des Quotienten aus dem Verlustmodul G" und dem Speichermodul G', der als tan δ oder auch als Verlusttangens bezeichnet wird) für das kommerzielle Produkt Durotak 87-625A ist in Figur 2 gezeigt.

Während das Produkt Durotak 87-625A als Polyisobutylen A gemäß der vorliegenden Erfindung bevorzugt ist, können selbstverständlich auch entsprechende Produkte anderer Hersteller oder des gleichen Herstellers verwendet werden, bei denen die Temperaturabhängigkeit des Speichermoduls im erfindungsgemäßen Bereich liegt.

In der haftklebenden Matrixschicht des erfindungsgemäßen transdermalen therapeutischen Systems liegt das Polyisobutylen A im Gemisch mit einem zweiten Polyisobutylen vor, das hier als Polyisobutylen B bezeichnet wird.

Auch das Polyisobutylen B wird durch sein Speichermodul G' gekennzeichnet. Anders als bei dem Polyisobutylen A muss bei dem Polyisobutylen B das Speichermodul im Bereich von 10 bis 40°C, vorzugsweise von 10 bis 60°C und insbesondere von 0 bis 80°C kontinuierlich fallen, und der Wert für das Speichermodul G' des Polyisobutylens B ist bei der niedrigeren Temperatur des jeweiligen Bereichs deutlich höher als bei der höheren Temperatur des jeweiligen Bereichs. Das Speichermodul G' des Polyisobutylens B ist in der Regel bei 0°C mindestens doppelt so hoch (absolut) wie bei 80°C, bevorzugt ist das Speichermodul G' bei 0°C mindestens fünf Mal so hoch (absolut) wie bei 80°C und besonders bevorzugt ist das Speichermodul G' des Polyisobutylens B bei 0°C mindestens 10 Mal so hoch (absolut) wie bei 80°C. In der Regel ist auch bei dem Polyisobutylen B das Speichermodul G' bei einer Temperatur von 10°C mindestens doppelt so hoch (absolut), bevorzugt mindestens dreimal so hoch (absolut) wie bei einer Temperatur von 40°C.

Der Absolutwert des Speichermoduls G' bei 0°C ist bei dem Polyisobutylen B bevorzugt im Bereich von 5 × 10⁴ Pascal bis 5 × 10⁶ Pascal, insbesondere im Bereich von 5 × 10⁴ Pascal bis 10⁶ Pascal und ganz besonders bevorzugt im Bereich von 10⁵ bis 10⁶ Pascal. Der Absolutwert des Speichermoduls G' bei 80°C ist bei dem Polyisobutylen bevorzugt im Bereich von 5 × 10² Pascal bis 5 × 10⁴ Pascal, stärker bevorzugt im Bereich von 5 × 10² Pascal bis 10⁴ Pascal und insbesondere im Bereich von 10³ bis 10⁴ Pascal.

Der Absolutwert des Speichermoduls G' ist bei den Polyisobutylen B bei 10°C, bevorzugt im Bereich von 2×10⁴ Pascal bis 10⁶ Pascal, insbesondere im Bereich von 5×10⁴ Pascal bis 10⁶ Pascal. Der Absolutwert des Speichermoduls G' bei 40°C ist bei dem Polyisobutylen B bevorzugt im Bereich von 5×10³ Pascal bis 2×10⁵ Pascal, stärker bevorzugt im Bereich von 10⁴ Pascal bis 10⁵ Pascal. Der Absolutwert des Speichermoduls G' bei 60°C ist bei dem Polyisobutylen B bevorzugt im Bereich von 5×10² Pascal bis 10⁵ Pascal, stärker bevorzugt im Bereich von 5×10³ Pascal bis 5×10⁴ Pascal. Wie beim Polyisobutylen A ist auch für das Polyisobutylen B die Molekulargewichtsverteilung für die vorliegende Erfindung nicht relevant, so lange das Speichermodul G' die erfindungsgemäß erforderliche Temperaturabhängigkeit aufweist.

Weiterhin hat sich gezeigt, dass zur Lösung der erfindungsgemäßen Ausgabe derartige transdermale therapeutische Systeme bevorzugt sind, die ein bestimmtes Verhältnis von Speichermodul zu Verlustmodul aufweisen. Bei dieser bevorzugten Ausführungsform können die erfindungsgemäßen transdermalen therapeutischen Systeme zusätzlich durch den Wert des Verlusttangens δ bei 30°C angegeben werden. So ist bei dem Polyisobutylen A der Verlusttangens δ bei 30°C, vorzugsweise im Bereich von 10⁻² bis 5×10⁻¹, stärker bevorzugt im Bereich von 5×10⁻² bis 5×10⁻¹. Bei den erfindungsgemäßen Polyisobutylenen B ist der Verlusttangens δ bei 30°C bevorzugt im Bereich von mehr als 5×10⁻¹ bis 10, insbesondere im Bereich von 6×10⁻¹ bis 5. Bevorzugt ist also in erfindungsgemäßen transdermalen therapeutischen Systemen der Verlusttangens bei dem Polyisobutylen B bei 30°C höher als bei dem Polyisobutylen A.

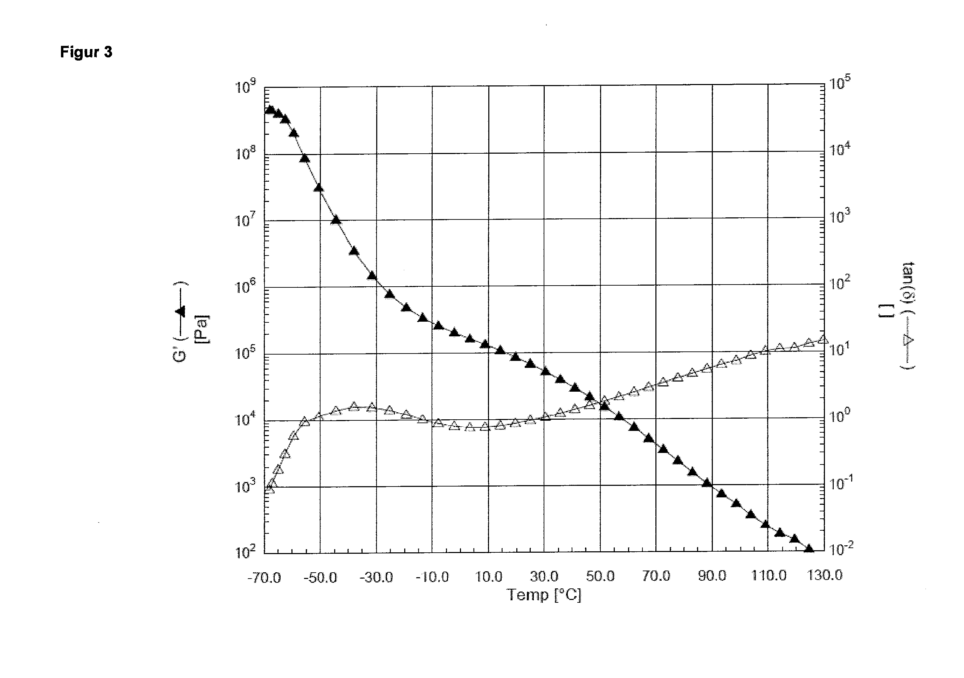

Wie das Polyisobutylen A kann auch das Polyisobutylen B beispielsweise durch Mischen von zwei oder mehr Polyisobutylenen hergestellt werden, so dass sich ein Polyisobutylen mit dem gewünschten Verlauf für das Speichermodul G' ergibt. Vorzugsweise handelt es sich bei Polyisobutylen B um ein einzelnes Polyisobutylen. Ein Beispiel für ein geeignetes Polyisobutylen B ist das Produkt Durotak 87-626A der Firma Henkel Corporation, Bridgewater, USA. Der Verlauf des Speichermoduls G' (zusammen mit dem Verlauf des Quotienten aus dem Verlustmodul G" und dem Speichermodul G', der als tan δ (oder auch als Verlusttangens) bezeichnet wird), für das kommerzielle Produkt Durotak 87-626A ist in Figur 3 gezeigt.

Während das Produkt Durotak 87-626A als Polyisobutylen B gemäß der vorliegenden Erfindung bevorzugt ist, können selbstverständlich auch entsprechende Produkte anderer Hersteller oder des gleichen Herstellers verwendet werden, bei denen die Temperaturabhängigkeit des Speichermoduls im erfindungsgemäßen Bereich liegt.

Da die haftklebende Matrixschicht des erfindungsgemäßen transdermalen therapeutischen Systems ein Gemisch des Polyisobutylens A und des Polyisobutylens B enthält und das Polyisobutylen A und/oder das Polyisobutylen B bevorzugt aus jeweils einem Polyisobutylen oder jeweils aus einem Gemisch aus zumindest zwei Polyisobutylenen mit unterschiedlichem mittleren Molekulargewicht besteht, kann die haftklebende Matrix des erfindungsgemäßen transdermalen therapeutischen Systems zwei, drei, vier oder mehr Polyisobutylene mit unterschiedlichem mittleren Molekulargewicht aufweisen.

Sofern im Rahmen der vorliegenden Beschreibung von einem mittleren Molekulargewicht die Rede ist, wird hierunter ein gewichtsgemitteltes mittleres Molekulargewicht verstanden, sofern nichts anderes ausdrücklich erwähnt ist oder sich aus den Umständen ergibt.

Erfindungsgemäß wird davon ausgegangen, dass ein einzelnes Polyisobutylen eine Molekulargewichtsverteilung mit einem einzelnen Peak ohne Schulter aufweist, die derjenigen Molekulargewichtsverteilung entspricht, die man bei der Polymerisation des Polyisobutylens aus den einzelnen Monomeren erhält. Werden zwei durch einfache Polymerisation hergestellte Polyisobutylene unterschiedlichen Molekulargewichts gemischt, liegt erfindungsgemäß ein Gemisch aus zwei Polyisobutylenen vor. Ein Gemisch aus zwei Polyisobutylenen ist erfindungsgemäß dadurch gekennzeichnet, dass die Molekulargewichtsverteilung des Gemisches aus zwei Polyisobutylenen zwei Peaks bei zwei unterschiedlichen Molekulargewichten aufweist oder die Molekulargewichtsverteilung einen Peak mit einer Schulter zeigt (falls die mittleren Molekulargewichte der gemischten Polyisobutylene zu nahe aneinander liegen, so dass sich keine zwei getrennten Peaks zeigen).

Bei dem erfindungsgemäßen transdermalen therapeutischen System besteht die haftklebende Matrixschicht aus einem Gemisch aus einem Polyisobutylen A und einem Polyisobutylen B, die sich durch die Temperaturabhängigkeit ihrer Speichermodule unterscheiden. In der Regel weist das Polyisobutylen A auch ein anders mittleres Molekulargewicht auf als das Polyisobutylen B. In der Regel wird daher die Molekulargewichtsverteilung des Gemisches aus dem Polyisobutylen A und dem Polyisobutylen B mindestens zwei Peaks aufweisen, von denen einer auf das Polyisobutylen A und einer auf das Polyisobutylen B zurückgeht. Es ist auch möglich, dass das Polyisobutylen A und/oder das Polyisobutylen B wiederum aus mindestens zwei Polyisobutylenen mit unterschiedlichem Molekulargewicht hergestellt wurde. In dem erfindungsgemäßen transdermalen therapeutischen System ist die haftklebende Matrixschicht aber bevorzugt aus zwei Polyisobutylenen aufgebaut, wobei jedes Polyisobutylen in der haftklebenden Matrixschicht des erfindungsgemäßen transdermalen therapeutischen Systems einen gesonderten Peak oder eine Schulter in der Molekulargewichtsverteilung zeigt.

Für den Fall, dass es sich bei Polyisobutylen A oder B jeweils um Gemische aus Polyisobutylen handelt, werden die einzelnen Polyisobutylene, aus denen das Polyisobutylen A und das Polyisobutylen B besteht, so gewählt, dass man jeweils die gewünschte Temperaturabhängigkeit des Speichermoduls für Polyisobutylen A bzw. B erhält.

Die Molekulargewichtsverteilung eines Polyisobutylens bzw. eines Gemisches mehrerer Polyisobutylene kann im Stand der Technik durch Gelpermeations-Chromatographie (GPC), beispielsweise gegen einen Polystyrol-Standard, bestimmt werden.

Die Menge an Polyisobutylen B in der haftklebenden Matrixschicht im Verhältnis zur Menge an Polyisobutylen A in der haftklebenden Matrixschicht kann von einem Fachmann geeignet eingestellt werden. In der Regel liegt das Gewichtsverhältnis von Polyisobutylen A : Polyisobutylen B in der Matrixschicht im Bereich von 10% (A) : 90% (B) bis 60% (A) : 40% (B), bevorzugt 20% (A) : 80% (B) bis 50% (A) : 50% (B), stärker bevorzugt 20% (A) : 80% (B) bis 40% (A) : 60% (B) und insbesondere 25% (A) : 75% (B) bis 35% (A) : 65% (B), jeweils bezogen auf das Gesamtgewicht der beiden Polyisobutylene, und bevorzugt ist aber der Gehalt an Polyisobutylen B in der Matrixschicht höher als der Gehalt an Polyisobutylen A. Die Menge an Polyisobutylen A in der Matrixschicht ist bevorzugt im Bereich von 10 bis 25%, stärker bevorzugt im Bereich von 10 bis 20%, insbesondere im Bereich von 12 bis 20%, insbesondere von 16 bis 18%, jeweils als Gewichtsprozent bezogen auf das Gesamtgewicht der Matrixschicht. Der Gehalt an Polyisobutylen B in der Matrixschicht liegt bevorzugt im Bereich von 30 bis 60%, stärker bevorzugt im Bereich von 30 bis 50% und insbesondere im Bereich von 40 bis 45%, jeweils als Gewichtsprozent bezogen auf das Gesamtgewicht der Matrixschicht.

Die Matrixschicht des erfindungsgemäßen transdermalen therapeutischen Systems enthält den Wirkstoff sowie das Polyisobutylen A und das Polyisobutylen B. Weiterhin enthält die Matrixschicht bevorzugt noch einen Permeationsverstärker. Als Permeationsverstärker kann ein beliebiger im Stand der Technik bekannter Permeationsverstärker für den Wirkstoff Fentanyl verwendet werden. Besonders bevorzugt wird erfindungsgemäß ein Carbonsäureester und hier insbesondere ein Fettsäureester verwendet. Besonders bevorzugt sind der Myristinsäureisopropylester (Isopropylmyristat) und der Ölsäureoleylester (Oleyloleat). Überraschenderweise hat sich gezeigt, dass bei Verwendung der erfindungsgemäßen Matrix der Permeationsverstärker Isopropylmyristat besonders gut geeignet ist, um ein transdermales therapeutisches System mit den gewünschten Eigenschaften bereitzustellen.

Der Gehalt an Permeationsverstärker in der Matrixschicht des erfindungsgemäßen transdermalen therapeutischen Systems ist nicht besonders eingeschränkt, er liegt aber in der Regel im Bereich von 2 bis 20 Gew.-% bezogen auf das Gesamtgewicht der Matrixschicht, besonders bevorzugt im Bereich von 5 bis 15 Gew.-%, beispielsweise bei 10 Gew.-% bezogen auf das Gesamtgewicht der Matrixschicht. Bei Verwendung von Isopropylmyristat als Permeationsverstärker hat sich der Bereich von 8 bis 15 Gew.-%, bezogen auf das Gesamtgewicht der erfindungsgemäßen Matrixschicht, als besonders vorteilhaft herausgestellt.

Weiterhin bevorzugt enthält die haftklebende Matrixschicht des erfindungsgemäßen transdermalen therapeutischen Systems auch noch einen Klebrigmacher. Als Klebrigmacher eignet sich insbesondere ein Polybuten, es kann aber auch ein anderer für Polyisobutylene geeigneter Klebrigmacher wie Rosinharz, Terpenharz oder Petrolharz verwendet werden. Polybuten ist als Klebrigmacher jedoch bevorzugt, insbesondere Polybuten mit einem zahlengemittelten Molekulargewicht im Bereich von 700 bis 6000, insbesondere von 900 bis 4000, wie das Produkt Indopol H-1900 mit einem zahlengemittelten mittleren Molekulargewicht von 2500. Bei Polybuten handelt es sich um ein Isobutylen/buten Copolymer.

Weiterhin ist als Klebrigmacher ein hydriertes oder nicht-hydriertes Kolophoniumharz, insbesondere ein hydriertes Kolophoniumharz, bevorzugt. Als Beispiel hierfür kann das kommerzielle Produkt Foral^{®} 105-E der Firma Eastman Chemical Middelburg BV, Den Haag, Niederlande genannt werden.

Der Gehalt an Klebrigmacher, insbesondere an Polybuten in der Matrixschicht des erfindungsgemäßen transdermalen therapeutischen Systems beträgt in der Regel 0 bis 40 Gew.-%, vorzugsweise 5 bis 35 Gew.-%, insbesondere 10 bis 30 Gew.-%, z.B. etwa 25 Gew.-%, jeweils bezogen auf das Gesamtgewicht der Matrixschicht.

Bei Verwendung von Isopropylmyristat als Permeationsverstärker hat es sich überraschenderweise als vorteilhaft herausgestellt, ein Polybuten mit einem zahlengemittelten Molekulargewicht im Bereich von 1800 bis 2800 zu verwenden, und zwar besonders vorteilhaft im Bereich von 22 bis 28 Gew.-%, bezogen auf das Gesamtgewicht der erfindungsgemäßen Matrixschicht.

Überraschenderweise hat sich zudem gezeigt, dass bei den erfindungsgemäß bevorzugten Mengenverhältnissen von Indopol 1900, Polyisobutylen A und Polyisobutylen B in dem transdermalen therapeutischen System der kalte Fluss bei typischen Lagertemperaturen von weniger als 20°C und nach Aufbringen auf die Haut (d.h. bei Temperaturen >30°C) besonders gering ist.

Weitere Bestandteile befinden sich erfindungsgemäß bevorzugt nicht in der Matrixschicht des erfindungsgemäßen transdermalen therapeutischen Systems. Die Matrixschicht des erfindungsgemäßen transdermalen therapeutischen Systems besteht daher erfindungsgemäß bevorzugt aus Wirkstoff, Polyisobutylen A, Polyisobutylen B, Permeationsverstärker (insbesondere ein Carbonsäureester, bevorzugt Isopropylmyristat oder Oleyloleat, besonders bevorzugt Isopropylmyristat) und Klebrigmacher (besonders bevorzugt Polybuten, insbesondere Indopol H-1900).

Die Matrixschicht der erfindungsgemäßen transdermalen therapeutischen Systeme ist insbesondere bevorzugt frei von Gelbildnern.

Die Matrix der erfindungsgemäßen transdermalen therapeutischen Systeme weist bevorzugt keinen verzweigten höherkettigen Alkohol, insbesondere bevorzugt überhaupt keinen höherkettigen Alkohol auf, wobei unter einem verzweigten höherkettigen Alkohol oder einem höherkettigen Alkohol ein Alkohol mit 5 oder mehr Kohlenstoffatomen verstanden wird.

In einer bevorzugten Ausführungsform weist das Polyisobutylen A beispielsweise ein viskositätsgemitteltes mittleres Molekulargewicht von ca. 1,100,000 g/mol und das Polyisobutylen B ein viskositätsgemitteltes mittleres Molekulargewicht von ca. 30.000 bis ca. 60.000 g/mol, vorzugsweise ca. 40.000 oder 55.000 g/mol auf. Bei dieser Ausführungsform enthält das Pflaster weiterhin einen Klebrigmacher und einen Permeationsverstärker, insbesondere den Klebrigmacher Indopol H 1900, also ein Polybuten mit einem zahlengemittelten Molekulargewicht von etwa 2500 g/mol und als Permeationsverstärker insbesondere entweder Oleyloleat oder Isopropylmyristat, wobei das Polybuten bevorzugt in einer Menge von 23 bis 28 Gew.-% vorhanden ist und das Isopropylmyristat bzw. das Oleyloleat in einer Menge von 8 bis 15 Gew.-% vorhanden ist.

In dem transdermalen therapeutischen System gemäß der Erfindung weist die Matrixschicht vorzugsweise eine Menge an Fentanyl oder einem Analogstoff davon auf, die ausreichend ist, um Schmerzfreiheit bei einem Menschen zu induzieren und für mindestens drei Tage, bevorzugt drei Tage aufrechtzuerhalten (bezogen auf den Zeitpunkt der Verabreichung des Pflasters). Ebenfalls bevorzugt ist, dass die Matrixschicht eine Menge an Fentanyl oder einen Analogstoff davon enthält, die ausreichend ist, um Schmerzfreiheit zu induzieren und für einen Zeitraum von mindestens drei Tagen, insbesondere von drei bis sieben Tagen, aufrechtzuerhalten.

Die absolute Menge an einzusetzendem Wirkstoff hängt von verschiedenen Faktoren, insbesondere von der Größe des einzusetzenden Pflasters und der Anwendungsdauer ab. Bevorzugt enthält das transdermale therapeutische System den Wirkstoff (insbesondere Fentanyl) in einer Menge von 3 bis 15 Gew.-%, besonders bevorzugt von 3 - 10 Gew.-%, insbesondere von 4 - 6 Gew.-% oder 5-6 Gew.-%, beispielsweise von etwa 6 Gew.-%, jeweils bezogen auf das Gesamtgewicht der Matrixschicht. In Abhängigkeit von der genauen Zusammensetzung der Matrixschicht und der verwendeten Menge an Wirkstoff kann der Wirkstoff entweder vollständig gelöst in der Matrixschicht vorliegen, oder es befinden sich einzelne Wirkstoffteilchen ungelöst in der Matrixschicht. In dem Fall, dass einzelne Wirkstoffteilchen sich noch ungelöst in der Matrixschicht befinden, spricht man von einem Suspensionspflaster. Ein Suspensionspflaster ist erfindungsgemäß bevorzugt und in Figur 1 gezeigt, wobei Bezugszeichen 4 die festen Wirkstoffteilchen in der Matrixschicht kennzeichnet.

Aus den vorstehenden Ausführungen ergeben sich bevorzugte Ausgestaltungen des erfindungsgemäßen transdermalen therapeutischen Systems, bei denen die Matrixschicht eine der in der nachstehenden Tabelle zusammengefassten Zusammensetzung aufweist.

| **BESTANDTEIL** | **GEW-%** | **GEW-%** | **GEW-%** | **GEW-%** | **GEW-%** |
|---|---|---|---|---|---|
| **Fentanyl** | 3-10 | 4-8 | 4-6 | 5.0-6.0 | 6 |
| **Permeationsverstärker** | 5-15 | 8-15 | 8-12 | 9-11 | 10 |
| **Polyisobutylen B** | 20-60 | 30-50 | 35-50 | 40-45 | 41,2 |
| **Polyisobutylen A** | 5-30 | 8-25 | 12-20 | 16-18 | 17,6 |
| **Klebrigmacher** | 15-45 | 15-40 | 20-35 | 23-28 | 25,2 |

| **BESTANDTEIL** | **GEW-%** | **GEW-%** | **GEW-%** | **GEW-%** | **GEW-%** |
|---|---|---|---|---|---|
| **Fentanyl** | 3-10 | 4-8 | 4-6 | 5.0-6.0 | 6 |
| **Isopropylmyristat** | 5-15 | 8-15 | 8-12 | 9-11 | 10 |
| **Polyisobutylen B** (besonders bevorzugt Durotak 87-626A) | 20-60 | 30-50 | 35-50 | 40-45 | 41,2 |
| **Polyisobutylen A** (besonders bevorzugt Durotak 87-625A) | 5-30 | 8-25 | 12-20 | 16-18 | 17,6 |
| **Polybuten** mit einem zahlengemittelten Molekulargewicht von etwa 2500 | 15-45 | 15-40 | 20-35 | 23-28 | 25,2 |

Die Gewichtsprozentangaben in der vorstehenden Tabelle beziehen sich jeweils auf das Gesamtgewicht der Matrixschicht. Das Flächengewicht der Matrixschicht liegt erfindungsgemäß bevorzugt im Bereich von 20 g/m² bis 100 g/m², insbesondere im Bereich von 30 g/m² bis 70 g/m², jeweils bezogen auf das Flächengewicht der getrockneten Matrixschicht.

Das erfindungsgemäße transdermale therapeutische System wird nach im Stand der Technik im Prinzip bekannten Verfahren hergestellt. Hierzu wird der Wirkstoff im Permeationsverstärker dispergiert. Das Polyisobutylen A und das Polyisobutylen B werden ebenfalls in einem geeigneten Lösemittel insbesondere einem flüssigen Alkan, insbesondere einen Alkan mit 5 bis 7 Kohlenstoffatomen, wie z.B. Heptan gelöst. Die gelösten Polyisobutylene werden unter Rühren mit dem dispergierten Fentanyl gemischt.

Anschließend wird gegebenenfalls der Klebrigmacher zugegeben und es wird weitergerührt, bis eine homogene Beschichtungsmasse entsteht. Die homogene Beschichtungsmasse wird dann auf die Abziehschicht aufgetragen und unter Erwärmen getrocknet, so dass der Restlösemittelgehalt möglichst gering ist. Bevorzugt beträgt der Restlösemittelgehalt weniger als 1%, insbesondere weniger als 0,5%. Die getrocknete Matrix wird mit der Rückschicht kaschiert, wodurch ein Laminat entsteht. Aus diesem Laminat werden Pflaster geeigneter Größe ausgestanzt.

Die hier beschriebenen transdermalen therapeutischen Systeme können nach dem vorstehenden erfindungsgemäßen Verfahren hergestellt werden und die Erfindung betrifft daher auch das Verfahren zur Herstellung der erfindungsgemäßen transdermalen therapeutischen Systeme und die nach diesem Verfahren erhältlichen transdermalen therapeutischen Systeme.

Weiter wurde erfindungsgemäß gefunden, dass folgendes transdermales System mit einer haftklebenden Matrixschicht auf Basis eines speziellen Polyacrylats ebenfalls die erfindungsgemäßen ausgezeichneten Eigenschaften, insbesondere die Freisetzung, aufweist.

Die vorliegende Erfindung betrifft damit in dieser Ausführungsform ein transdermales therapeutisches System zur Verabreichung eines Wirkstoffs durch die Haut, umfassend oder bestehend aus
a) einer Rückschicht,
b) eine haftklebende Matrixschicht, die den Wirkstoff enthält, und
c) eine Abziehschicht (Release-Liner),
wobei der Wirkstoff Fentanyl oder ein Analogstoff des Fentanyl ausgewählt aus Alfentanil, Lorfentanil, Lofentanil, Remifentanil und Trefentanil oder ein Salz einer dieser Wirkstoffe ist, bevorzugt Fentanyl und vorzugsweise in einer Menge von 8 Gew.-% bis 12 Gew.-%, bezogen auf das Gesamtgewicht der Matrixschicht, mehr bevorzugt etwa 10 Gew.-% in der Matrixschicht enthalten ist, wobei die Matrixschicht als haftklebendes Polymer ein Acrylatvinylacetat Copolymer in einer Menge von ca. 82-88 Gew.-%, vorzugsweise etwa 85 Gew.-%, bezogen auf das Gesamtgewicht der Matrixschicht, sowie etwa 5 Gew.-% eines Polymer-Regulators, vorzugsweise PVP, enthält, wobei die haftklebende Matrixschicht keinen ungelösten Wirkstoff enthält und worin das Acrylat-Vinylacetat Copolymer aus etwa 68% 2-Ethylhexylacrylat, etwa 27% Vinylacetat und etwa 5% 2-Hydroxyethylacrylat sowie gegebenenfalls geringen Mengen (< 0.5%) Glycidylmethacrylat als Vernetzer aufgebaut ist (alle %-Angaben als Gewichtsprozent). Bevorzugt sind die Handelsprodukte DURO-TAK 87-2287, DURO-TAK 87-4287 oder DURO-TAK 87-3916. Bevorzugt wird als PVP das kommerzielle Produkt Polyvidon 25 verwendet.

Das transdermale therapeutische System gemäß der Ausführung basierend auf einer Matrixschicht auf Basis des speziellen Polyacrylats entspricht bezüglich des Aufbaus aus Rückschicht (1), Matrixschicht (2), gegebenenfalls der vor Gebrauch zu entfernenden Abdeckschicht (3) und Abziehschicht der oben beschriebenen Ausführungsform des transdermalen therapeutischen Systems mit einer Matrixschicht auf Basis eines Polyisobutylens. Die oben beschriebenen bevorzugten Ausführungsformen für die Rückschicht (1), Abziehschicht (3) gelten entsprechend für das transdermale System enthaltend eine Matrixschicht auf Basis eines Polyacrylats. Auch können die oben beschriebenen Permeationsverstärker, insbesondere Isopropylmyristat oder Oleyloleat in den oben genannten Mengen eingesetzt werden.

Das folgende Beispiel erläutert die Erfindung.

Ein transdermales therapeutisches System mit einer haftklebenden Matrixschicht der folgenden Zusammensetzung:
- 6% Fentanyl
- 10% Isopropylmyristat
- 41,2% Durotak 87-626A
- 17,6% Durotak 87-625A und
- 25,2% Indopol 1900
wurde wie folgt hergestellt.
0,6 g Fentanyl wurden in 1,0 g Isopropylmyristat dispergiert. Der Ansatz wurde bis zur homogenen Suspension gerührt. 5,58 g Durotak 87-626A (Feststoffgehalt 73,71%) und 14,48 g Durotak 87-625A (Feststoffgehalt 12,18%) wurden unter Rühren über einen Zeitraum von 20 Minuten zu dem Ansatz aus Fentanyl und Isopropylmyristat zugegeben. Anschließend wurden 2,52 g Polybuten zu dem restlichen Gemisch zugefügt.

Es wurde gerührt, bis eine homogene Beschichtungsmasse entsteht. Diese homogene Beschichtungsmasse wurde auf einer Folie der Marke Scotchpak 9742 in einer Dicke von 117 µm aufgetragen. Das Gemisch wurde anschließend stufenweise auf 50°C, 60°C, 70°C und 80°C erwärmt und getrocknet. Das Flächengewicht der getrockneten Matrixschicht beträgt ca. 50 g/m². Die getrocknete Matrix wurde mit einer Rückschicht der Marke Scotchpak 9723 kaschiert. Aus dem Laminat wurden Pflasterfragmente in geeigneter Größe ausgestanzt.

Die so hergestellten Pflaster wurden 8 Wochen im Kühlschrank bei 2°C bis 8°C, bei 25°C und 60% relativer Luftfeuchtigkeit und bei 40°C und 75% relativer Luftfeuchtigkeit gelagert. Bei den Pflastern trat kein nennenswerter kalter Fluss auf. Bei allen untersuchten Pflastern unter allen untersuchten Bedingungen lag der maximale kalte Fluss unter 0,3 mm. Als Vergleich wurde ein transdermales therapeutisches System gemäß Beispiel 2 der WO 2011/029948 hergestellt und über 8 Wochen bei 25°C/60% Luftfeuchtigkeit und bei 40°C/75% Luftfeuchtigkeit gelagert. Die Vergleichspflaster zeigten einen ausgeprägten kalten Fluss an der Abziehhilfe und auch an der Rückschicht, der wesentlich größer war als der kalte Fluss, der sich bei dem erfindungsgemäßen Pflaster zeigte.

Mit den erfindungsgemäßen Pflastern wurden *in vitro*-Studien durchgeführt. Es zeigten sich Permeationsraten in Franz-Zellen, die im Wesentlichen denen des Pflasters des Beispiels 2 der WO 2011/029948 entspricht.

Die Pflaster haften ohne nennenswerte Hautreizungen und werden lokal bis zu drei Tagen auf der Haut gut vertragen.

Damit zeigen die erfindungsgemäßen transdermalen therapeutischen Systeme die gleiche gute Hautverträglichkeit, die gleiche Klebkraft und gleiche Permeationsraten wie gängige kommerzielle Systeme. Anders als die transdermalen therapeutischen Systeme der WO 2011/029948 zeigen die erfindungsgemäßen transdermalen therapeutischen Systeme aber bei der Lagerung praktisch keinen kalten Fluss und sind auch wesentlich einfacher und kostengünstiger mit noch geringerem Wirkstoffgehalt herzustellen.

## Patentansprüche

1. Transdermales therapeutisches System zur Verabreichung eines Wirkstoffs durch die Haut umfassend oder bestehend aus
a) eine Rückschicht,
b) eine haftklebende Matrixschicht, die den Wirkstoff enthält, und
c) eine Abziehschicht (Release-Liner),
wobei der Wirkstoff Fentanyl oder ein Analogstoff des Fentanyls ausgewählt aus Alfentanil, Carfentanil, Lofentanil, Remifentanil und Trefentanil oder ein Salz eines dieser Wirkstoffe ist und
wobei die Matrixschicht als haftklebendes Polymer ein Gemisch aus einem Polyisobutylen A und einem Polyisobutylen B enthält,
wobei das Polyisobutylen A ein Speichermodul G' aufweist, dessen Wert im Temperaturbereich von 10°C bis 40°C im Wesentlichen konstant ist und
wobei das Polyisobutylen B ein Speichermodul G' aufweist, dessen Wert im Temperaturbereich von 10°C bis 40°C mit steigender Temperatur kontinuierlich abnimmt,
wobei das Speichermodul G' im linearen viskoelastischen Bereich bei einer Frequenz von 10 rad/sec unter Verwendung eines Rheometers mit paralleler Plattengeometrie und parallelen Platten gemessen wird, und
wobei die haftklebende Matrixschicht ungelösten Wirkstoff in Form von Wirkstoffpartikeln enthält.

2. Transdermales therapeutisches System nach Anspruch 1, wobei es sich bei dem Wirkstoff um Fentanyl handelt.

3. Transdermales therapeutisches System nach Anspruch 1 oder 2, wobei für das Polyisobutylen A im Temperaturbereich von 10°C bis 40°C alle Werte des Speichermoduls G' von dem Wert für das Speichermodul G' bei 40°C um nicht mehr als 50%, bevorzugt um nicht mehr als 25% abweichen.

4. Transdermales therapeutisches System nach einem der vorstehenden Ansprüche, wobei für das Polyisobutylen B das Speichermodul G' bei 10°C mindestens das 2-fache, bevorzugt mindestens das 3-fache des Speichermoduls G' bei 80°C beträgt.

5. Transdermales therapeutisches System nach einem der vorstehenden Ansprüche, wobei der Gehalt des Polyisobutylens A zum Polyisobutylen B in der Matrixschicht im Bereich von 20% (A) : 80% (B) bis 40% (A) : 60% (B), bevorzugt im Bereich von 25% (A) : 75% (B) bis 35% (A) : 65% (B) liegt, jeweils bezogen auf das Gesamtgewicht der Polyisobutylene A und B.

6. Transdermales therapeutisches System nach einem der vorstehenden Ansprüche, wobei es sich bei dem Polyisobutylen A und bei dem Polyisobutylen B jeweils um einzelne Polyisobutylene mit unterschiedlichen mittleren Molekulargewichten handelt.

7. Transdermales therapeutisches System nach einem der Ansprüche 1-6, wobei die Matrixschicht einen Permeationsverstärker enthält, bei dem es sich bevorzugt um Isopropylmyristat oder Oleyloleat handelt.

8. Transdermales therapeutisches System nach einem der vorstehenden Ansprüche, wobei die Matrixschicht einen Klebrigmacher (tackifier) enthält, bei dem es sich bevorzugt um ein Polybuten oder einen hydrierten oder nicht hydrierten Kolophoniumester handelt.

9. Transdermales therapeutisches System nach einem der vorstehenden Ansprüche, **dadurch gekennzeichnet, dass** die Wirkstoffmenge für eine Applikationsdauer von 3 Tagen ausreichend ist und der Wirkstoff in der Matrixschicht in einer Konzentration im Bereich von 3-15 Gew.-%, vorzugsweise im Bereich von 4-6 Gew.-% und am bevorzugtesten im Bereich von 5-6 Gew.-% vorhanden ist (bezogen auf das Gewicht der Matrixschicht).

10. Transdermales therapeutisches System nach einem der vorstehenden Ansprüche, **dadurch gekennzeichnet, dass** in der haftklebenden Matrixschicht neben dem Wirkstoff, dem Polyisobutylen A und dem Polyisobutylen B nur noch ein Klebrigmacher, bevorzugt ein Polybuten oder ein hydrierter Kolophoniumester und ein Permeationsverstärker, bevorzugt Isopropylmyristat oder Oleyloleat vorhanden ist.

11. Transdermales therapeutisches System nach Anspruch 10, bei dem der Klebrigmacher in einer Menge im Bereich von 23 bis 28% und der Permeationsverstärker in einer Menge im Bereich von 8 bis 15 % vorhanden sind, jeweils bezogen auf das Gesamtgewicht der Matrixschicht.

12. Verfahren zur Herstellung eines transdermalen therapeutischen Systems nach einem der Ansprüche 1-11, bei dem der Wirkstoff im Permeationsverstärker dispergiert wird,
das Polyisobutylen A und das Polyisobutylen B, die wie in Anspruch 1, 3, 4 oder 6 definiert sind, jeweils in einem geeigneten Lösungsmittel verteilt werden, anschließend die beiden polymerhaltigen Lösungen homogen gemischt werden,
die polymerhaltigen Lösungen mit dem dispergierten Wirkstoff und gegebenenfalls weitere Bestandteile gemischt werden, bis eine einheitliche Masse entsteht,
die so entstandene Masse auf die Abziehschicht oder die Rückschicht aufgebracht wird, das Lösungsmittel entfernt wird,
die Rückschicht bzw. die Abziehschicht auflaminiert wird und
transdermale therapeutische Systeme der gewünschten Größe ausgeschnitten oder ausgestanzt werden.

13. Transdermales therapeutisches System nach einem der Ansprüche 1-11, erhältlich nach einem Verfahren nach Anspruch 12.
